# EUROPÄISCHE PATENTSCHRIFT

(11) **EP 2 172 234 B1**
(45) Veröffentlichungstag und Bekanntmachung des Hinweises auf die Patenterteilung: **22.10.2014**
(21) Anmeldenummer: 09169483.6
(22) Anmeldetag: 04.09.2009
(51) Int. Cl.: A61L 31/02, A61L 31/14

(54) **Implantat sowie Verfahren zur Herstellung einer degradationshemmenden Schicht auf einer Körperoberfläche eines Implantats**
Implant and method for creating a degradation-inhibiting coating on the surface of a body of an implant
Implant et procédé de fabrication d'une couche réduisant la dégradation sur la surface d'un implant

(30) Priorität: 06.10.2008 DE 102008042603
(43) Veröffentlichungstag der Anmeldung: 07.04.2010
(73) Patentinhaber: Biotronik VI Patent AG, 6341 Baar (CH)
(72) Erfinder: Bayer, Ullrich, 18211, Admannshagen-Bargeshagen (DE); Becher, Bärbel, 18057, Rostock (DE); Block, Bernd, 18055, Rostock (DE)
(74) Vertreter: Lindner-Vogt, Karin L.

(56) Entgegenhaltungen:
- EP-A2- 1 941 918
- WO-A1-2008/045184
- WO-A2-2004/060318
- DE-A1-102007 004 589
- US-A1- 2004 254 608
- DATABASE WPI Week 199550 Thomson Scientific, London, GB; AN 1995-390577 XP002699641, & JP H07 268380 A (PENTEL KK) 17. Oktober 1995 (1995-10-17)
- DATABASE WPI Week 197809 Thomson Scientific, London, GB; AN 1978-16706A XP002699642, & JP S53 5264 A (TOYO RUBBER CHEM IND CO) 18. Januar 1978 (1978-01-18)
- DATABASE EPODOC [Online] EUROPEAN PATENT OFFICE, THE HAGUE, NL; XP002699643, & JP H09 241861 A (DAIHATSU MOTOR CO LTD) 16. September 1997 (1997-09-16)

## Beschreibung

Die Erfindung betrifft ein Verfahren zur Herstellung einer degradationshemmenden ersten Schicht auf einer Körperoberfläche eines Implantats, insbesondere einer intraluminalen Endoprothese, wobei der Körper mindestens ein zumindest weitgehend biodegradierbares metallisches Material aufweist, sowie ein Implantat erhältlich oder erhalten durch ein derartiges Verfahren.

Medizinische Endoprothesen oder Implantate für die unterschiedlichsten Anwendungen sind in großer Vielfalt aus dem Stand der Technik bekannt. Als Endoprothesen im Sinne der vorliegenden Erfindung sind endovaskuläre Prothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen ein rohrförmiges oder hohlzylinderförmiges Grundgitter auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderer Implantate kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für das Grundgitter biodegradierbarer Implantate geeignete Werkstoffe können beispielsweise Polymere oder Metalle enthalten. Das Grundgitter kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff zumindest teilweise ein Metall enthält, vorzugsweise Magnesium oder eine Magnesiumlegierung.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Als für den genannten Zielkorridor der Degradation besonders vielversprechend haben sich biodegradierbare Magnesium-Implantate, insbesondere Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass sich bei Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemische Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

Ein weiteres Problem im Zusammenhang mit Passivierungsbeschichtungen ergibt sich daraus, dass Stents oder auch andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert bzw. (bei Verwendung einer Formgedächtnislegierung als Implantatmaterial) beispielsweise durch Erwärmung über eine Sprungtemperatur in den expandierten Zustand überführt. Aufgrund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen ergibt sich somit der Nachteil, dass die Beschichtung während der Verformung des Implantats reißt (z.B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Aus der Druckschrift DE 10 2006 060 501 ist ein Verfahren zur Herstellung einer korrosionshemmenden Beschichtung auf einem Implantat aus einer biokorrodierbaren Magnesiumlegierung sowie ein nach dem Verfahren erhältliches Implantat bekannt, bei dem nach Bereitstellen des Implantats die Implantatoberfläche mit einer wässrigen oder alkoholischen Konversionslösung enthaltend ein oder mehrere Ionen ausgewählt aus der Gruppe K⁺, Na⁺, NH₄⁺, Ca²⁺, Mg²⁺, Zn²⁺, Ti⁴⁺, Zr⁴⁺, Ce³⁺, Ce⁴⁺, PC₃³⁻, PO₄³⁻, HPO₄²⁻, H₂PO₄⁻, OH⁻, BO₃³⁻, B₄O₇³⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻, wobei eine Konzentration des Ions oder der Ionen jeweils im Bereich von 10⁻² mol/l bis 2 mol/l liegt, behandelt wird. Die Behandlung der Implantatoberfläche mit der genannten Konversionslösung bedingt eine anodische Oxidation des Implantats. Sie wird entweder ohne Verwendung einer äußeren Stromquelle (außen stromlos) oder mit einer Stromquelle durchgeführt. Die in dieser Druckschrift beschriebenen Verfahrensbeispiele sowie Elektrolytzusammensetzungen erfüllen jedoch nicht die Erwartungen hinsichtlich Degradationsverhalten und Dilatationsfähigkeit ohne Schichtzerstörung bei der Anwendung für einen Magnesium-Stent.

Die Aufgabe der vorliegenden Erfindung besteht somit darin, ein Verfahren zur Herstellung einer degradationshemmenden ersten Schicht auf einem Körper eines Implantats anzugeben, welche eine Degradation des Implantats im gewünschten Zielkorridor ermöglicht. Dabei soll die Degradation zu einem kontrollierbaren Zeitpunkt stattfinden und zusätzlich die Dilatation bzw. Verformung des Implantats ohne nennenswerten Einfluss auf das Degradationsverhalten ermöglichen. Entsprechend besteht die Aufgabe der Erfindung außerdem darin, ein derartiges Implantat zu schaffen.

Die obige Aufgabenstellung wird gelöst durch ein Verfahren umfassen die folgenden Schritte:
a) Bereitstellen des Körpers des Implantats,
b) Aufbringen der ersten Schicht auf mindestens einen Teil der Körperoberfläche, wobei die erste Schicht Magnesiumstearat enthält, dadurch gekennzeichnet, dass vor dem Aufbringen der ersten Schicht mindestens ein Teil der Körperoberfläche einer plasmachemischen Behandlung in einer wässrigen Lösung zur Erzeugung einer Zwischenschicht unterzogen wird, bei der eine das Plasma erzeugende elektrische Spannung an den Körper des Implantats angelegt wird, wobei die wässrige Lösung ein oder mehrere Ionen ausgewählt aus der Gruppe der Phosphate, Carbonate und Silikate aufweist und/oder Sr²⁺-Ionen enthält, wobei die Sr²⁺-Ionen vorzugsweise in einer Konzentration von jeweils etwa 0,05 Mol/l bis etwa 2,0 Mol/l Sr²⁺ in der wässrigen Lösung enthalten sind.

Hierbei umfasst der Körper des Implantats mindestens einen Teil des Implantats, vorzugsweise den Hauptteil des Implantats, welcher die mechanische Integrität des Implantats bewirkt.

Mittels des erfindungsgemäßen Verfahrens kann ein Implantat hergestellt werden, das sich durch Defektfreiheit der Körperoberfläche durch nachträgliches Versiegeln mit der ersten Schicht auszeichnet. Lokale Fehlstellen und/oder in dem Körper des Implantats vorhandene Poren werden wirksam vor dem Kontakt mit korrosiv wirkenden Körperflüssigkeiten geschützt. Die hydrophobe Oberflächeneigenschaft und der geringe Kristallwassergehalt des Magnesiumstearats, der auch durch einen vorzugsweise durchgeführten, sich an das Aufbringen der ersten Schicht anschließenden Trocknungsschritt erzeugt wird, bewirken eine äußerst geringe Diffusion von Wasser zum Grundwerkstoff des Implantat-Körpers. Sowohl die an der Oberfläche des Implantats vorhandenen, herstellungsbedingten lokalen Kontaminationen als auch die durch die Legierungszusammensetzung des Implantat-Körpers an der Oberfläche liegenden Ausscheidungen werden durch das Magnesiumstearat inert eingebettet und können somit nicht mehr mit den Umgebungsbedingungen reagieren. Ebenso wird das Loslösen von Partikeln mit geringer Bindungsneigung von der Oberfläche des Implantat-Körpers beim Dilatieren verhindert. Diese Partikel verbleiben in der zähen, hoch flexiblen Magnesiumstearatschicht. Hierdurch ergibt sich eine erhöhte Hämo- beziehungsweise Biokompatibilität.

Durch die mittels des erfindungsgemäßen Verfahrens hergestellte erste Schicht vereinfachen sich zudem die Lage- und Transportbedingungen der nach dem erfindungsgemäßen Verfahren hergestellten Implantate, da die Stabilität eines solchen Implantats gegenüber Degradation höher ist als bei unbeschichteten Implantaten.

Aufgrund der Beschichtung des Implantat-Körpers mit der ersten Schicht wird in vorteilhafter Weise bewirkt, dass der Reibungskoeffizient des Implantats sinkt. Hieraus folgt, dass beim Verschieben beispielsweise eines Stents als Implantat in einem Katheter geringere Kräfte aufgewendet werden müssen. Dadurch wird im Falle eines Stents eine genauere Stentfixation ermöglicht. Zudem werden das Crimpen und die spätere Freisetzung des Implantats an dem zu behandelnden Ort vereinfacht.

In einem bevorzugten Ausführungsbeispiel des erfindungsgemäßen Verfahrens wird die erste Schicht mittels Eintauchen in eine Lösung aufgebracht, wobei die Lösung Magnesiumstearat und ein Lösungsmittel, vorzugsweise Aceton und/oder Isopropanol, enthält und vorzugsweise eine Temperatur zwischen etwa 10°C und 40°C aufweist. Alternativ kann die Magnesiumstearatschicht auch derart aufgebracht werden, dass die genannte magnesiumstearathaltige Lösung auf den Körper des Implantats aufgesprüht wird (spray coating). Dabei wird das Teil in einer Kammer an einem dünnen Draht aufgehängt und mittels einem rotierenden Teller (Chargenhalter) allseitig besprüht.

In einem bevorzugten Ausführungsbeispiel kann die Effektivität des Tauchprozesses durch Anlegen eines Drucks erfolgen, der kleiner als der Umgebungsdruck, vorzugsweise kleiner als etwa 90% des Umgebungsdrucks ist, d.h. des Luftdrucks an dem Ort, an dem der Tauchprozess durchgeführt wird. Der hierbei eintretende Entgasungseffekt führt zu einer schnellen Füllung der filigranen Oberflächenstruktur des Implantats mit Magnesiumstearat.

Es ist weiter von Vorteil, wenn der Implantat-Körper vor der Beschichtung mit Magnesiumstearat eine raue Oberfläche aufweist. Hierdurch wird die Haftung der ersten Schicht auf dem Implantat-Körper verbessert.

Nach einer Verweildauer von einigen Minuten in der Lösung, vorzugsweise von mindestens etwa 2 Minuten, wird der beschichtete Körper aus dem Tauchbad entfernt und im Trockenofen bei einer Temperatur, die größer ist als die Raumtemperatur, vorzugsweise größer als etwa 30°C, getrocknet. Hierbei ist es besonders bevorzugt, wenn die Trocknungstemperatur möglichst gering ist, d.h. zwischen etwa 40°C und etwa 70°C liegt, da hierdurch ein langsames Freisetzen/Abdampfen des mindestens einen Lösungsmittels führt, wodurch einen porenfreie erste Schicht, die Magnesiumstearat enthält, erzeugt wird.

Die Effektivität der Magnesiumstearatbeschichtung kann beispielsweise durch Verschieben des Implantats in einer rohrförmigen Testvorrichtung, beispielsweise ein Stent in einem Katheter, nachgewiesen werden. Die Magnesiumstearatbeschichtung bewirkt, dass bei einer Verschiebung des Implantats in der Testvorrichtung geringere Kräfte aufgebracht werden müssen. Des Weiteren kann der durch das Magnesiumstearat erreichte Versiegelungseffekt mittels in vitro Auslagerung in künstlichem Plasma nachgewiesen werden. Die Versiegelung durch die Magnesiumstearatbeschichtung bewirkt eine geringere Abnahme des metallischen Querschnitts während der Auslagerung gegenüber einem Implantat ohne Beschichtung.

Bei der vorliegenden Erfindung wird vor dem Aufbringen der ersten Schicht und nach dem Bereitstellen des Körpers des Implantats mindestens ein Teil der Körperoberfläche einer plasmachemischen Behandlung zur Erzeugung einer Zwischenschicht unterzogen. Die plasmachemische Behandlung umfasst dabei die Behandlung in einem wässrigen Elektrolytsystem (wässrige Lösung), bei der plasmachemische Effekte direkt an der Oberfläche des Körpers des Implantats entstehen. Das für Mikrosekunden stabile Plasma an der Körperoberfläche erzeugt Reaktionsprodukte, welche zur Ausbildung der Zwischenschicht auf der Körperoberfläche führen. Die Zwischenschicht wird vorzugsweise derart auf einem entsprechenden Teil oder der gesamten Körperoberfläche des Implantats angeordnet, dass die Zwischenschicht zwischen der Körperoberfläche und der ersten Schicht, die Magnesiumstearat enthält, liegt.

Durch die plasmachemische Behandlung bilden sich auf der Körperoberfläche des Implantats Phosphate, Hydroxide und Oxide des metallischen Materials, insbesondere des Magnesiums. Diese Schichtzusammensetzung stellt bei Kontakt mit der Körperflüssigkeit einen temporären Korrosionsschutz dar, der eine verzögerte Degradation des metallischen Materials bewirkt. Die im Zuge der verzögerten Degradation des Implantats frei werdenden Partikel werden teilweise von körpereigenen Zellen inkorporiert und/oder weiter abgebaut. Die mittels des erfindungsgemäßen Verfahrens hergestellte degradationshemmende Schicht weist verfahrensbedingt Poren auf, welche zunächst naturgemäß korrosive Schwachstellen bilden, durch die dem Elektrolyt der Kontakt zum metallischen Grundmaterial erleichtert wird. Die vor und nach den Degradationstest durchgeführten oberflächenanalytischen Untersuchungen haben jedoch gezeigt, dass die mit der plasmachemischen Behandlung einhergehende Hydroxidbildung zu einer unten beschriebenen lokal begrenzten Versiegelung der Porengründe führt.

Die Versiegelung der Porengründe kann beispielsweise anhand des mehrstufigen Verlaufs des pH-Werts eines Elektrolyten in einem Korrosionstest (zum Beispiel künstliches Plasma) nachgewiesen werden. In den ersten sieben Tagen steigt der pH-Wert erwartungsgemäß von 7,4 auf 8,5 bis 8,7 an. Nach einem nach sieben Tagen durchgeführten Medienwechsel erhöht sich der pH-Wert in überraschender Weise von 7,4 nur noch auf 8,0. Dieses Verhalten zeigt eine geringere chemische Aktivität des metallischen Grundmaterials (insbesondere des Magnesiums) mit dem korrosiven Medium. Dieser Effekt geht einher mit einem Stopp der Querschnittsverjüngung des Materials des Implantat-Körpers, der bereits nach sieben Tagen im metallographischen Querschliff zu verzeichnen ist. Dieser Effekt kann beispielsweise dadurch erklärt werden, dass Hydroxide des metallischen Materials die Poren in der Zwischenschicht ausfüllen und damit der Kontakt des Elektrolyten zum metallischen Grundmaterial unterbunden wird. Durch diesen Selbstheilungseffekt wird die Standzeit der Implantate unter Körpermilieubedingungen wesentlich erhöht. Außerdem kann die Degradationsgeschwindigkeit durch Variation der Schichtdicke gesteuert werden. Hierdurch eröffnet sich auch die Möglichkeit, die Degradationsdauer des Implantats an den spezifischen Implantationsort anzupassen (koronar, intercranial, renal etc.) Ein weiterer Vorteil des plasmachemischen Prozesses besteht darin, dass die durch die vorgeschalteten Oberflächenbehandlungsprozesse des Implantats nicht entfernbaren O-berflächenkontaminationen des Grundmaterials durch die degradationshemmende Zwischenschicht absorbiert werden und somit den Degradationsprozess nicht noch zusätzlich negativ beeinflussen. Zudem werden aus der Oberfläche herausragende, teilweise scharfkantige Ausscheidungen des Implantat-Körpers (die zum Beispiel nicht gelöste Legierungsbestandteile, z. B. Yttrium und dessen Verbindungen, enthalten) abgedeckt. Daraus ergibt sich eine noch weiter erhöhte Hämo- bzw. Biokompatibilität.

Außerdem weist die verfahrensbedingt poröse Struktur der Zwischenschicht ein hohes plastisches Verformungsvermögen auf. Beispielsweise werden die beim Dilatieren eines Stents entstehenden Mikrorisse durch Energieakkumulation beziehungsweise -dissipation in den zu den Mikrorissen benachbarten Poren gestoppt. Es kommt somit nicht zu einer Delamination der Zwischenschicht.

In der wässrigen Lösung sind ein oder mehrere Ionen ausgewählt aus der Gruppe der Phosphate, Carbonate, Hydroxide und Silikate enthalten. In einem besonders bevorzugten Ausführungsbeispiel enthält die wässrige Lösung Sr²⁺-Ionen, die vorzugsweise in einer Konzentration von jeweils etwa 0,05 Mol/l bis etwa 2,0 Mol/l Sr²⁺-Ionen in der wässrigen Lösung enthalten sind. Hierdurch werden Strontiumverbindungen in die Zwischenschicht, d.h. die Oberflächenschicht, des Implantats eingelagert. Dies ist vorteilhaft, da insbesondere Strontiumcarbonat kaum wasserlöslich ist und somit einen die Degradation besonders hemmende Schichtbestandteil in der Oberflächenschicht des Implantats bildet. Zudem kann das in der Beschichtung enthaltene Strontiumcarbonat insbesondere bei cranialen Anwendungen eine arzneimittelähnliche Wirkung gegen Zerebralsklerose entfalten.

Das mittels plasmachemischer Beschichtung behandelte Implantat wird anschließend in einem Lösungsmittel, vorzugsweise destilliertem H₂O, gespült und danach vorzugsweise bei einer Temperatur von mindestens etwa 80°C, besonders bevorzugt mindestens etwa 100°C getrocknet, wobei die Trocknung vorzugsweise in einem Umluftofen durchgeführt wird.

Um den pH-Wert des Elektrolyten (wässrigen Lösung) konstant zu halten, ist bevorzugt in der wässrigen Lösung ein Puffer, vorzugsweise Kaliumdihydrogenphosphat und/oder Natriumdihydrogenphosphat enthalten.

In einem weiteren bevorzugten Ausführungsbeispiel wird der Implantat-Körper vor der plasmachemischen Behandlung elektrochemisch behandelt, vorzugsweise elektrochemisch poliert. Hierdurch werden Verunreinigungen auf der Oberfläche des Implantat-Körpers entfernt, so dass die plasmachemische Behandlung an einer definierten Oberfläche erfolgt.

Bevorzugt erfolgt die plasmachemische Behandlung des Implantat-Körpers dadurch, dass an den Körper eine gepulste, positive Spannung angelegt wird, deren Amplitude im überwiegenden Zeitraum der Behandlung mindestens etwa 90 Volt übersteigt, besonders bevorzugt mindestens etwa 100 Volt übersteigt und welche vorzugsweise im Verlauf der Behandlung ansteigt. Durch diese hohen gepulsten Spannungen mit einer Pulslänge von vorzugsweise maximal etwa 20 Mikrosekunden, besonders bevorzugt etwa 5 Mikrosekunden, werden an der Oberfläche des Implantat-Körpers über Mikrosekunden Plasmen erzeugt, welche zur Reaktion des metallischen Materials des Implantatkörpers mit dem Elektrolyten führen. Zwischen den Spannungspulsen folgt eine Ruhephase von vorzugsweise ca. 100 Mikrosekunden.

Bevorzugt wird der plasmachemische Prozess mit einer Stromdichte von mindestens etwa 8 mA/cm² vorzugsweise mindestens etwa 10 mA/cm² durchgeführt.

In einem weiteren bevorzugten Ausführungsbeispiel kann auf die erste Schicht eine zweite Schicht, vorzugsweise eine Schicht bestehend zumindest überwiegend aus Parylene, aufgetragen werden. Bevorzugte Schichtdicken der Parylene-Schicht liegen zwischen etwa 0,1 und 10 µm. Durch eine derartige Schichtkombination kann die Degradationszeit des Implantats nochmals wesentlich erhöht werden. Hierbei ist Parylene die Bezeichnung für vollständig lineare, teilkristalline, unvernetzt aromatische Polymere. Die verschiedenen Polymere besitzen unterschiedliche Eigenschaften und lassen sich in vier Grundtypen einteilen, nämlich Parylene C, Parylene D, Parylene N und Parylene F. Insbesondere bei Verwendung der Parylene-N-Variante wird ein sehr gleichmäßiges Degradationsbild erreicht. Dies bedeutet, dass die Oberflächenkorrosion bei der in einem bevorzugten Ausführungsbeispiel verwendeten Kombination einer Magnesiumstearatschicht, der plasmachemisch erzeugten Schicht und einer zweiten Schicht mit Parylene N dem gewünschten Degradationszielkorridor am nächsten kommt.

Die obige Aufgabenstellung wird ferner gelöst durch ein Implantat erhältlich durch das oben beschriebene erfindungsgemäße Verfahren, welches zumindest auf einem Teil seiner Oberfläche eine erste Schicht aufweist, die Magnesiumstearat enthält.

Die erfindungsgemäßen Implantate weisen die oben im Zusammenhang mit dem erfindungsgemäßen Herstellungsverfahren angegebenen Vorteile auf.

In der vorliegenden Erfindung beträgt die Dicke der ersten Schicht 0,5 µm bis 10 µm, vorzugsweise etwa 1,0 µm bis etwa 5,0 µm. Die Konzentration des Magnesiumstearates in der ersten Schicht liegt dabei etwa zwischen 80 Gew.% und 100 Gew.%. Insbesondere ist es hinsichtlich der Biokompatibilität der ersten Schicht vorteilhaft, wenn die Schicht so lange getrocknet und damit entgast wird, bis die Lösungsmittel restlos verdampft sind.

In der vorliegenden Erfindung weist die mittels der plasmachemischen Behandlung erzeugte erste Zwischenschicht eine Dicke von 1 bis 20 µm, vorzugsweise etwa 1 bis 8 µm auf. Eine Schicht mit einer Dicke von etwa 2 bis 5 µm ist aufgrund des höheren Plastifizierungsvermögens zu favorisieren.

Oben wurde bereits beschrieben, dass die Zwischenschicht Poren aufweist, wobei vorzugsweise auf dem Porengrund ein Hydroxid des metallischen Materials oder der metallischen Materialien des Implantat-Körpers gebildet ist. Durch das auf dem Porengrund angeordnete Hydroxid wird eine lokal begrenzte Versiegelung des Porengrunds erreicht.

Bevorzugt enthält die Zwischenschicht mindestens eine Verbindung ausgewählt aus der Gruppe Phosphate, Hydroxide und Oxide des biodegradierbaren Materials oder der biodegradierbaren Materialien, Strontiumkarbonat, Strontiumphosphat.

Das erfindungsgemäße Verfahren wird nachfolgend anhand eines Beispiels erläutert.

Zunächst wird ein Implantat in Form eines Stents mit einem Körper bestehend aus einer Magnesiumlegierung, vorzugsweise WE43 (93 Gew.% Magnesium, 4 Gew.% Yttrium (W) und 3 Gew.% Seltenerdmetalle (E) außer Yttrium) bereitgestellt.

Anschließend wird eine wässrige Lösung (Elektrolyt) dargestellt. Hierzu wird zunächst in ein Becherglas 500 ml destilliertes H₂O gefüllt. Danach werden in der nachfolgend angegebenen Reihenfolge die Komponenten der wässrigen Lösung hinzugegeben. Hierbei ist darauf zu achten, dass die nächstfolgende Komponente erst nach völliger Auflösung der vorangegangenen Komponenten zugegeben werden darf. Der Elektrolyt ist bei der Zugabe ständig zu rühren, was beispielsweise mit einem Magnetrührer (500 min⁻¹) realisiert werden kann. Die Zugabe der Komponenten sollte außerdem langsam erfolgen, um eine übermäßige Wärmeentwicklung zu vermeiden. Alternativ kann die Elektrolytsynthese auch in einem doppelwandigen, kühlbaren Behälter erfolgen. Folgende Komponenten können zugegeben werden
1. 100 ml/l Ethylendiamin-Lösung (99%), 100 g/l Kaliumdihydrogenphosphat, 90 g/l Strontiumhydroxid-Octahydrat und 40 ml/l wässrige Ammoniumhydroxidlösung (25%)
   oder
2. 50 ml/l Ethylendiamin-Lösung (99%), 50 g/l Kaliumdihydrogenphosphat, 90 g/l Strontiumnitrat und 20 ml/l wässrige Ammoniumhydroxidlösung (25 Gew.%) oder 25 g/l Natriumhydroxid.

Folgende Zusammensetzungen können alternativ für eine plasmachemische Beschichtung in einer wässrigen Lösung ohne Sr²⁺ - Ionen gewählt werden:

| | Ethylendiamin-Lösung (99%) | Kaliumdihydrogenphosphat | wässrige Ammonium-hydroxidlösung (25%) | Natriumhydroxid |
|---|---|---|---|---|
| 3. | 30 ml/l | 10 g/l | 10 ml/l | 0 |
| 4. | 30 ml/l | 10 g/l | 0 | 10 g/l |

Nach der Fertigstellung einer der vier mit 1., 2., 3. oder 4. bezeichneten Ansätze folgt der plasmachemische Beschichtungsprozess.

Vor dem plasmachemischen Beschichtungsprozess kann ggf., je nach Kontaminationszustand des Implantat-Körpers, ein mehrstufiges Entfetten in tensidhaltigen Lösungen und anschließendes Spülen in destilliertem H₂O erfolgen.

Vor Beginn des plasmachemischen Beschichtungsprozesses wird der Körper des Implantats elektrisch kontaktsicher mittels eines Titan- oder eines Aluminiumdrahts mit der Anode von Elektroden verbunden. Die Gegenelektrode (Kathode) besteht aus einem rost- und säurebeständigen Stahl. Anode und Kathode der Elektroden werden mit einer Spannungsquelle verbunden, welche in der Lage ist, eine gepulste Spannung abzugeben. Die Stromdichte beträgt ca. 10 mA/cm².

Nach dem Eintauchen des Implantat-Körpers in die wässrige Lösung wird eine ständig steigende, gepulste Badspannung angelegt. Beim Erreichen des für die Behandlung von Magnesiumlegierungen in der Elektrolytzusammensetzung charakteristischen Badspannungsbereichs von mehr als 100 V kommt es zu plasmachemischen Oberflächeneffekten. Diese erzeugen Oberflächenschichten, die sowohl aus Oxiden, Hydroxiden und Phosphaten des metallischen Grundmaterials des Implantat-Körpers als auch aus weiteren Verbindungen bestehen, die sich aus Elementen des Elektrolyten rekrutieren. Insbesondere wird aufgrund der Anwesenheit des Sr²⁺-Ions auch Strontiumkarbonat gebildet, das sich aus dem Kohlenstoff des Ethylendiamin, dem Sauerstoff des wässrigen Elektrolyten und dem Strontium des Strontiumhydroxides bzw. Strontiumnitrats zusammensetzt. Außerdem wird Strontiumphosphat gebildet, das ein plasmachemisches Umwandlungsprodukt des Kaliumdihydrogenphosphates und des Wassers ist.

Nach dem Erreichen der Beschichtungsendspannung von ca. 250 V sinkt die vorher eingestellte Stromdichte von ca. 10 mA/cm² auf ca. 6 mA/cm² ab. Wird dieser Wert erreicht, so wird die Stromzufuhr unterbrochen und der plasmachemische Prozess beendet. Die Schichtdicke der so erzeugten Zwischenschicht beträgt vorzugsweise etwa 1 µm bis etwa 20 µm, besonders bevorzugt zwischen 1 µm und 8 µm, und hängt wesentlich von der verwendeten Badspannung ab.

Anschließend erfolgt ein mehrstufiges Spülen des Implantatkörpers in destilliertem Wasser, eine Trennung des Implantats vom Kontaktdraht und eine Trocknung des Körpers des Implantats in einem Umluftofen bei ca. 100°C.

Nach dem plasmachemischen Beschichtungsprozess, der mit dem im vorangegangenen Abschnitt beschriebenen Trocknungsschritt abgeschlossen ist, wird der Implantat-Körper auf einen Kunststofffaden (z.B. Polyamid) aufgehängt und anschließend in die Lösung zur Auftragung des Magnesiumstearats getaucht. Die Lösung besteht z.B. aus 9 Teilen hochreinem Aceton oder Isopropanol und 1 Teil Magnesiumstearat. Der Tauchvorgang erfolgt bei Raumtemperatur in einem evakuierbaren Exsikkator. In diesem wird ein Unterdruck von ca. 100 mbar mittels einer Pumpe erzeugt. Hierdurch werden die filigranen und durch die vorangegangene plasmachemische Vorbehandlung entstandenen mikroporösen Oberflächenstrukturen bzw. die Hinterschneidungen und kompliziert geformten Strukturen effektiv von Restgas befreit. Dadurch kann in der Lösung eine vollständige Bedeckung der Stentoberfläche durch das Magnesiumstearat erfolgen, das auch in die Oberflächenstrukturen und Hinterschneidungen eindringt. Nach einer Verweildauer von etwa 3 Minuten in dem Tauchbad wird der Exsikkator belüftet, das Implantat aus dem Tauchbad entnommen und anschließend in einem Umluftschrank, immer noch am Kunststofffaden hängend, bei einer Temperatur von 60°C getrocknet. Die Schichtdicke der so erhaltenen Magnesiumstearat-Beschichtung liegt dabei im Bereich von etwa 0,5 bis etwa 10 µm.

Bedingt durch den im Exsikkator vorliegenden Unterdruck wird das Magnesiumstearat sehr gleichmäßig auf der Oberfläche abgeschieden. Eine geringe Trocknungstemperatur bewirkt in vorteilhafter Weise ein langsames Freisetzen/Abdampfen der Lösungsmittel der Tauch-Lösung, so dass eine porenfreie Magnesiumstearatschicht erhalten wird. Handelt es sich bei dem so hergestellten Implantat um einen Stent, so kann der mit der ersten Schicht und der Zwischenschicht versehene Körper anschließend mit einem Katheter komplettiert und einer Strahlensterilisation unterzogen werden.

In einem weiteren Ausführungsbeispiel kann die der Tauchbehandlung zur Erzeugung der Magnesiumstearatschicht vorangehende plasmachemische Behandlung mit anschließendem Spülen und Trocknen auch weggelassen werden.

Mittels des beschriebenen Verfahrens hergestellte Implantate degradieren in dem gewünschten Zeitfenster. Überraschender Weise lässt sich hierbei die Degradation u.a. durch eine Schicht (Zwischenschicht) steuern, welche porös ausgebildet ist.

## Patentansprüche

1. Verfahren zur Herstellung einer degradationshemmenden ersten Schicht auf einer Körperoberfläche eines Implantats, insbesondere einer intraluminalen Endoprothese, wobei der Körper mindestens ein zumindest weitestgehend biodegradierbares metallisches Material aufweist, umfassend die folgenden Schritte:
a) Bereitstellen des Körpers des Implantats,
b) Aufbringen der ersten Schicht auf mindestens einen Teil der Körperoberfläche, wobei die erste Schicht Magnesiumstearat enthält,
**dadurch gekennzeichnet, dass** vor dem Aufbringen der ersten Schicht mindestens ein Teil der Körperoberfläche einer plasmachemischen Behandlung in einer wässrigen Lösung zur Erzeugung einer Zwischenschicht unterzogen wird, bei der eine das Plasma erzeugende elektrische Spannung an den Körper des Implantats angelegt wird, wobei die wässrige Lösung ein oder mehrere Ionen ausgewählt aus der Gruppe der Phosphate, Carbonate und Silikate aufweist und/oder Sr²⁺-Ionen enthält, wobei die Sr²⁺-Ionen vorzugsweise in einer Konzentration von jeweils etwa 0,05 Mol/l bis etwa 2,0 Mol/l Sr²⁺ in der wässrigen Lösung enthalten sind.

2. Verfahren nach Anspruch 1, **dadurch gekennzeichnet, dass** die erste Schicht mittels Eintauchen in eine Lösung aufgebracht wird, die Magnesiumstearat und ein Lösungsmittel, vorzugsweise Aceton und/oder Isopropanol, enthält und vorzugsweise eine Temperatur zwischen etwa 10°C und 40°C aufweist.

3. Verfahren nach einem der vorhergehenden Ansprüche, **dadurch gekennzeichnet, dass** das Aufbringen der ersten Schicht bei einem Druck erfolgt, der kleiner als der Umgebungsdruck, vorzugsweise kleiner als etwa 90% des Umgebungsdrucks ist.

4. Verfahren nach einem der vorhergehenden Ansprüche, **dadurch gekennzeichnet, dass** der Körper des Implantats nach dem Aufbringen der ersten Schicht bei einer Temperatur von mindestens etwa 30°C, besonders bevorzugt bei einer Temperatur zwischen etwa 40°C und etwa 70°C getrocknet wird, wobei die Trocknung vorzugsweise in einem Umluftofen durchgeführt wird.

5. Verfahren nach einem der vorhergehenden Ansprüche, **dadurch gekennzeichnet, dass** in der wässrigen Lösung ein Puffer, vorzugsweise Kaliumdihydrogenphosphat und/oder Natriumdihydrogenphosphat enthalten ist.

6. Verfahren nach einem der vorhergehenden Ansprüche, **dadurch gekennzeichnet, dass** die Körperoberfläche vor der plasmachemischen Behandlung elektrochemisch behandelt, vorzugweise elektrochemisch poliert wird.

7. Verfahren nach einem der vorhergehenden Ansprüche, **dadurch gekennzeichnet, dass** die plasmachemische Behandlung der Körperoberfläche dadurch erfolgt, dass an den Körper eine gepulste Spannung angelegt wird, deren Amplitude im überwiegenden Teil des Behandlungszeitraums mindestens etwa 90 V übersteigt, besonders bevorzugt mindestens etwa 100 V übersteigt und vorzugsweise im Verlauf der Behandlung ansteigt.

8. Verfahren nach einem der vorhergehenden Ansprüche, **dadurch gekennzeichnet, dass** die Stromdichte bei der plasmachemischen Behandlung mindestens etwa 8 mA/cm², vorzugsweise mindestens etwa 10 mA/cm² beträgt.

9. Verfahren nach einem der vorhergehenden Ansprüche, **dadurch gekennzeichnet, dass** auf die erste Schicht eine zweite Schicht, vorzugsweise bestehend mindestens überwiegend aus Parylene, aufgebracht wird.

10. Implantat, insbesondere intraluminale Endoprothese, mit einem Körper, der mindestens ein zumindest weitestgehend biodegradierbares metallisches Material aufweist, erhältlich durch ein Verfahren gemäß einem der vorhergehenden Ansprüche.

11. Implantat, insbesondere intraluminale Endoprothese, mit einem Körper, der mindestens ein zumindest weitestgehend biodegradierbares metallisches Material aufweist, wobei der Körper zumindest auf einem Teil seiner Oberfläche eine erste Schicht aufweist, die Magnesiumstearat enthält und die Dicke der ersten Schicht 0,5 µm bis 10 µm, vorzugsweise etwa 1,0 µm bis etwa 5,0 µm beträgt, **dadurch gekennzeichnet, dass** die Dicke der mittels der plasmachemischen Behandlung auf der Körperoberfläche erzeugten Zwischenschicht 1 bis 20 µm, vorzugsweise etwa 2 bis etwa 8 µm beträgt und die Zwischenschicht mindestens eine Verbindung ausgewählt aus der Gruppe enthaltend Phosphate, Hydroxide und Oxide des biodegradierbaren metallischen Materials oder der biodegradierbaren metallischen Materialien, Strontiumkarbonat, Strontiumphosphat aufweist.

## Claims

1. A method for producing a degradation-inhibiting first layer on a surface of an implant body, in particular of an intraluminal endoprosthesis, wherein the body has at least one metallic material, which is at least largely biodegradable, comprising the following steps:
a) preparing the body of the implant,
b) applying the first layer to at least a portion of the body surface, wherein the first layer contains magnesium stearate,
**characterised in that**, prior to the application of the first layer, at least part of the body surface is subjected to a plasma-chemical treatment in an aqueous solution in order to produce an intermediate layer, in which an electric voltage generating the plasma is applied to the body of the implant, wherein the aqueous solution comprises one or more ions selected from the group of phosphates, carbonates and silicates and/or contains Sr²⁺ ions, wherein the Sr²⁺ ions are preferably contained in a concentration in each case from approximately 0.05 mol/l to approximately 2.0 mol/l Sr²⁺ in the aqueous solution.

2. The method according to Claim 1, **characterised in that** the first layer is applied by immersion into a solution containing magnesium stearate and a solvent, preferably acetone and/or isopropanol, and preferably having a temperature between approximately 10 °C and 40 °C.

3. The method according to one of the preceding claims, **characterised in that** the first layer is applied at a pressure which is lower than ambient pressure, preferaby lower than approximately 90 % of ambient pressure.

4. The method according to one of the preceding claims, **characterised in that** the body of the implant is dried at a temperature of at least approximately 30 °C, especially preferably at a temperature between approximately 40 °C and approximately 70 °C, after applying the first layer, wherein the drying is preferably performed in a circulating air oven.

5. The method according to one of the preceding claims, **characterised in that** a buffer, preferably potassium dihydrogen phosphate and/or sodium dihydrogen phosphate, is contained in the aqueous solution.

6. The method according to one of the preceding claims, **characterised in that** the body surface is treated electrochemically prior to the plasma-chemical treatment, preferably being electrochemically polished.

7. The method according to one of the preceding claims, **characterised in that** the plasma-chemical treatment of the body surface is performed by applying a pulsed voltage to the body, the amplitude exceeding at least approximately 90 V over most of the treatment period of time, especially preferably exceeding at least approximately 100 V and preferably rising during the course of the treatment.

8. The method according to one of the preceding claims, **characterised in that** the current density in the plasma-chemical treatment amounts to at least approximately 8 mA/cm², preferably at least approximately 10 mA/cm².

9. The method according to one of the preceding claims, **characterised in that** a second layer, preferably consisting at least predominantly of parylene, is applied to the first layer.

10. An implant, in particular an intraluminal endoprosthesis, with a body which has at least one metallic material that is at least largely biodegradable, obtainable by a method according to one of the preceding claims.

11. An implant, in particular an intraluminal endoprosthesis, with a body which has at least one metallic material which is at least largely biodegradable, wherein the body has a first layer over at least a portion of its surface, said layer containing magnesium stearate and the thickness of the first layer being 0.5 µm to 10 µm, preferably approximately 1.0 µm to approximately 5.0 µm, **characterised in that** the thickness of the intermediate layer produced on the body surface by means of the plasma-chemical treatment is 1 to 20 µm, preferably approximately 2 to approximately 8 µm, and the intermediate layer comprises at least one compound selected from the group containing phosphates, hydroxides and oxides of the biodegradable metallic material or the biodegrable metallic materials, strontium carbonate, strontium phosphate.

## Revendications

1. Procédé pour la fabrication d'une première couche anti-dégradation sur une surface de corps d'un implant, en particulier d'une endoprothèse intraluminale, dans lequel le corps comporte au moins un matériau métallique au moins très largement biodégradable, comprenant les étapes suivantes:
a) mise à disposition du corps de l'implant,
b) application de la première couche sur au moins une partie de la surface de corps, la première couche contenant un stéarate de magnésium,
**caractérisé en ce qu'**avant l'application de la première couche, au moins une partie de la surface de corps est soumise à un traitement chimique au plasma dans une solution aqueuse pour la production d'une couche intermédiaire, dans lequel une tension électrique produisant le plasma est appliquée au corps de l'implant, la solution aqueuse contenant un ou plusieurs ions sélectionnés parmi le groupe des phosphates, des carbonates et des silicates et/ou des ions Sr²⁺, les ions Sr²⁺ étant contenus dans la solution aqueuse de préférence selon une concentration respective d'environ 0,05 Mol/l à environ 2,0 Mol/l Sr²⁺.

2. Procédé selon la revendication 1, **caractérisé en ce que** la première couche est appliquée par immersion dans une solution contenant du stéarate de magnésium et un agent solvant, de préférence de l'acétone et/ou de l'isopropanol, et présentant de préférence une température comprise entre 10°C et 40°C.

3. Procédé selon l'une des revendications précédentes, **caractérisé en ce que** l'application de la première couche est réalisée avec une pression inférieure à la pression ambiante, de préférence inférieure à environ 90% de la pression ambiante.

4. Procédé selon l'une des revendications précédentes, **caractérisé en ce que** suite à l'application de la première couche, le corps de l'implant est séché à une température d'au moins environ 30°C, de façon particulièrement préférée à une température comprise entre environ 40°C et environ 70°C, le séchage étant réalisé de préférence dans un four à circulation d'air.

5. Procédé selon l'une des revendications précédentes, **caractérisé en ce que** la solution aqueuse contient un tampon, de préférence du dihydrogénophosphate de potassium et/ou du dihydrogénophosphate de sodium.

6. Procédé selon l'une des revendications précédentes, **caractérisé en ce qu'**avant le traitement chimique au plasma, la surface de corps est traitée électrochimiquement, de préférence polie électrochimiquement.

7. Procédé selon l'une des revendications précédentes, **caractérisé en ce que** le traitement chimique au plasma de la surface de corps est réalisé en appliquant une tension pulsée au corps, dont l'amplitude dépasse au moins environ 90 V pendant la majeure partie de la durée de traitement, dépasse de façon particulièrement préférée au moins environ 100 V, et augmente de préférence au cours du traitement.

8. Procédé selon l'une des revendications précédentes, **caractérisé en ce que** la densité du courant pendant le traitement chimique au plasma s'élève à au moins environ 8 mA/cm², de préférence à au moins environ 10 mA/cm².

9. Procédé selon l'une des revendications précédentes, **caractérisé en ce qu'**une deuxième couche, de préférence constituée au moins majoritairement de parylène, est appliquée sur la première couche.

10. Implant, en particulier une endoprothèse intraluminale, avec un corps comportant au moins un matériau métallique au moins très largement biodégradable, lequel peut être obtenu à l'aide d'un procédé selon l'une des revendications précédentes.

11. Implant, en particulier une endoprothèse intraluminale, avec un corps comportant au moins un matériau métallique au moins très largement biodégradable, dans lequel le corps comporte une première couche contenant un stéarate de magnésium sur au moins une partie de sa surface, l'épaisseur de la première couche étant comprise entre 0,5 µm et 10 µm, de préférence entre environ 1,0 µm et environ 5,0 µm, **caractérisé en ce que** l'épaisseur de la couche intermédiaire produite au moyen du traitement chimique au plasma sur la surface de corps est comprise entre 1 et 10 µm, de préférence entre environ 2 et environ 8 µm, et la couche intermédiaire comporte au moins un composé sélectionné parmi le groupe comprenant des phosphates, des hydroxydes et des oxydes du matériau métallique biodégradable ou des matériaux métalliques biodégradables, le carbonate de strontium, le phosphate de strontium.
